# EUROPEAN PATENT SPECIFICATION

(11) **EP 0 627 444 B1**
(45) Date of publication and mention of the grant of the patent: **10.06.1998**
(21) Application number: 94830111.4
(22) Date of filing: 16.03.1994
(51) Int. Cl.: C07K 5/083, A61K 38/06

(54) **Monoacetic derivatives of the peptides leu-phe-ala and ala-leu-phe and their use in identifying and destroying tumour cells, viruses and virus-carrying cells**
Monoessigsäure Derivate der Peptide leu-phe-ala und ala-leu-phe und deren Verwendung in der Erkennung und Vernichtung von Tumorzellen und virusinfizierten Zellen
Dérivés monoacétiques des peptides leu-phe-ala et ala-leu-phe et leur utilisation à la reconnaissance et la destruction des cellules de tumeur et des cellules infectées par des virus

(30) Priority: 22.03.1993 EP 93830113
(43) Date of publication of application: 07.12.1994
(73) Proprietor: Grippa, Leonida, I-00154 Rome (IT)
(72) Inventor: Grippa, Leonida, I-00154 Rome (IT)
(74) Representative: Banchetti, Marina

(56) References cited:
- EP-A- 0 174 245
- EP-A- 0 282 374
- DE-A- 3 200 273

## Description

The present invention concems the monoacetic derivatives of the tripeptides leucine-phenylalanine-alanine and alanine-leucine-phenylalanine, and their use in the fight against tumours and viruses.

In Italian patent applications Nos. 94691A/78 and 51885A/78, filed by the same applicant, there are disclosed some methods for obtaining mixtures of substances or pure substances capable of enhancing the defensive ability of the body through the physiologic stimulation of cells which take part in mechanisms of immunologic nature occurring in the body itself.

In Italian patent No. 1148005, filed by the same applicant and corresponding to DE-A-3 200 273, a tripeptide is disclosed whose six possible combinations are capable of enhancing the T-lymphocytes' immune activity; thus, such a tripeptide behaves as an immunomodulator.

The complex mechanism of the immune system, with its effector cells and their products (i.e., immunoglobulins, lymphokines, monokines, etc.), appears to be a big biochemical complex, in which the different components, that stimulate and repress, activate and inactivate, produce and eliminate, are in a dynamic balance, showing continuous changes depending upon the relative amounts of humoral and cellular components making up such a complex. As a consequence, every time the relative amounts of the components of the system are either physiologically or artificially modified, the system reacts by activating humoral and cellular powers, that counteract the altered components for the purpose of attaining a new balance for the whole system. This explains why everything tried from an immune stand-point by means of immunoproteins, interferons, interleukines, adoptive immunotherapies, etc. failed to bring about those results which "in vitro" tests had allowed to postulate.

In fact, as far as aberrant neoplastic cells are concerned, it is not possible to grant any chance of survival to even only one of them; therefore, the impact of a system (such as the immune system), that involves a final balance with the consequent presence - inside the system itself - of vital aberrant cells also, is undoubtedly favourable to the latter cells.

It is known that among the various groups of specific lymphocytes (i.e., helper, suppresser, killer, etc.), there is also a small group (about 10% of all circulating lymphocytes) found in all mammals, that seems to come from a primordial immunosurveillance system. The lymphocytes of this group are able to eliminate, in a non-specific way, that is without the need for identifying in advance a definite antigen, the clones of aberrant cells that have been transformed, even those transformed into neoplastic elements.

The lymphocytes that are supposed to be in charge of such a type of surveillance are NK cells (i.e., natural killer cells) and form the first line of a non-specific defence, which is clearly differentiated from that made by the immune system by means of other lymphocytic populations (i.e., T4 - T8, etc.).

NK cells, genotypically different from T cells, are a population endowed with a cytotoxic and macrophagic activity, whose cells are the most ancient elements entrusted with the defence of the body, and were present before the development of the immunocompetent cells of the immune system. Such a cell population has maintained its primordial characteristics even when the other T cells (thymus-dependent) underwent those morphological and biochemical changes which are at the basis of those sophisticated tasks that they carry out within the immune system.

Owing to these primordial features, NK cells have maintained over the time a mechanism for manifesting a direct cytotoxic activity, not mediated by any immune mechanism, and they appear to be certainly involved with the task of forming the first immunosurveillance line.

In fact, by acting in an autonomous way, without the need to identify any special antigen, NK cells escape the balance mechanisms of the immune system, thus exerting their cytotoxic action until obtaining the final destruction of the last cell present in the tissues.

The significance of this first aspecific line of defence appears to be manifest, even if up to date we know neither how it is activated nor how it is connected with the other great specific line of defence, namely the immune system.

In the frame of the preliminary studies which led to the present invention, in order to discover whether and how NK cells' cytotoxic ability could be activated in a physiological way, it was first observed that NK lymphocytes have always exerted their cytotoxic and macrophagic activity within another system, namely the inflammatory system. The latter is a mechanism preceding the development of the immune system and even at the present time it is used by the body both for reparative processes taking place in its tissues and for defensive purposes.

Therefore, NK lymphocytes are cells that had to maintain their morphological and biochemical characteristics (during body development) inasmuch as they participated in the inflammatory process; moreover, they had to develop additional features, with special reference to immunochemical characteristics, in order to be able to participate with their cytotoxic activity in the immune process, whose evolution took place following the development of the inflammatory process.

Therefore, we may suppose that, in addition to IL 2R receptor, NK cells possess on their membrane also a receptor capable of blocking a compound suitable for functioning as a growth and differentiation factor for this cellular clone, to which it confers also the ability of uptaking the immuno-antibodies that are then exposed quickly on its membrane.

According to the present invention, a peptide released during inflammatory processes leading to tissue cicatrization was identified in the applicant's laboratories. Such a peptide was isolated and then synthesized again in its possible isomers.

Subsequently, in the first phase it was studied the possibility that the monoacetic derivatives of the various isomers of the peptide, following their inoculation into normoergic (germ free) mice, could increase the number of NK lymphocytes and, consequently, also their cytotoxic capacity.

In the second phase it was studied the possibility that the activated NK lymphocytes could exert their incremented cytotoxic activity against either viruses, bacteria or clones of aberrant cells (including tumour cells) without the need for identifying a definite antigen. In fact, as it has been pointed out before, the possibility of a direct activation, by means of these peptide derivatives, of the cytotoxic activity of NK lymphocytes, thus excluding the path of the immune system connected with the antigen cycle, would result in the advantage of an immediate and final lymphocytic action (that is, till the destruction of the last extraneous organism or aberrant cell present in tissues).

As a result of a number of tests, some of which are described further on, it has been established that the monoacetic derivatives of two different tripeptides, namely leucine-phenylalanine-alanine and alanine-leucinephenylalanine, possess the property of activating NK cells to identify and destroy directly, that is without any interference by the immune system, aberrant tumour cells, metastatic cells, viruses and virus-carrying cells. The activation mechanism, whose existence was never suspected, nor it is reported in the international literature, is believed to follow the modalities hereinafter reported, which are the expression of a physiologic "active immunotherapy".

It is possible to postulate a pattern similar to that involved during the inflammatory process: a few viruses or a few aberrant cells initially present in a body tissue induce the release of a peptide that immediately contacts zonal lymphocytes by anchoring itself to a specific receptor placed on their membrane. The interaction between the peptide (which is probably carried by a particular protein) and the receptor activates the intracellular mechanisms that signal to NK cells to move into action. Thus, the said interaction appears to be the sign that controls the growth and differentiation of NK cells.

Once activated, NK cells increment their cytotoxic property and, as a consequence, they block the extraneous material and neutralize it without requiring any intervention by the immune system.

However, when this first line of defence, due either to an inadequate response, such as for a low number of NK lymphocytes present, or to any other cause, results to be unable to overcome the danger involving the body, then the more complex and sophisticated immune system begins to manifest its supporting activity.

The immune response follows its course; together with the development of specific antibodies against the viruses or the aberrant clones, cytotoxic T-lymphocytes become activated as well, and they exert their action in the presence of immuno-antibodies, thus effectively supporting the cytotoxic action exerted by NK lymphocytes.

The importance of the first defence line made up by NK lymphocytes lies in the fact that almost for sure they are the only elements that carry out the true immunological surveillance, whereas the immune system has the task to activate a complete and organized defence. The NK lymphocytes do not allow for aberrant clones, that are initially isolated from each other, to reproduce and organize themselves to such an extent as to result ultimately successful over the sophisticated apparatus of the immune system when said system is activated.

It appears logical, therefore, to postulate that it is actually the defeat of the first defence line to prompt the body to develop the neoplasm, which is thus the winner over the immune system.

Therefore, the remarkably interesting aspect of the action exerted by the investigated peptides on NK lymphocytes, with their consequent activation, lies in the fact that, by resorting to such a mechanism, a clonal expansion of the NK cells and an increase in their cytotoxic capacity are induced, which in turn bring about, in the organisms that are infected with a virus or are affected by a tumour, a physiological "active immunotherapy" (different from the "adoptive immunotherapy" that may be obtained by inoculating LAK and IL 2 cells).

Thus, this type of "active immunotherapy" may result to be winning both over clones of disorganized (metastatic) tumour cells and over virus-carrying cells (such as, e.g., in AIDS cases), especially where T-lymphocytes, activated by an immune process, are unsuccessful.

Accordingly, the present invention provides the monoacetic derivatives (i.e., the N-monoacetylated amides) of the tripeptides leucine-phenylalanine-alanine and alanine-leucine-phenylalanine, and their use in the fight against tumours, in particular against metastatic neoplastic cells, and in the treatment and prophylaxis of viral diseases, i.e. in the fight against viruses and virus-carrying cells.

The invention also provides pharmaceutical compositions comprising as active ingredient the monoacetic derivative of a tripeptide chosen between leucine-phenylalanine-alanine and alanine-leucine-phenylalanine or a mixture thereof, in a pharmaceutically acceptable carrier. Said pharmaceutical compositions are primarily intended for use in the treatment or prophylaxis of cancer, and in the treatment or prophylaxis of viral diseases.

The compounds of the instant invention can be obtained by first synthesizing the relevant tripeptides by the solid phase peptide synthesis according to Merrifield's outline.

According to such a scheme, the synthesis of a peptide (in this case a tripeptide) may be carried out when the end of the peptide chain is anchored to an insoluble support, and therefore it is in a solid phase. Following the attainment of the desired tripeptide sequence, in view of the fact that such a sequence is still bound to the solid support, a reagent must be used for the purpose of separating the peptide chain from the support, thus releasing the synthetized peptide into the final solution.

The solid support is a synthetic polymer possessing some reactive groups, which are able to react with the carboxyl radical of the amino acid, so that ultimately the latter is bound to the polymer by a covalent bond. The protective group on the amino moiety must be suitable for being removed readily and without any damage to the bond linking the amino acid to the support. Following removal of this protective moiety, a second amino acid, protected on the NH₂ group, may be allowed to react by its COOH group with the NH₂ group of the first amino acid in order to form the first dipeptide. When the same procedure is repeated by using a third amino acid the desired tripeptide is obtained. At the end of this synthesis a specific reagent is used in order to break the bond that ties the first amino acid to the polymer, thus releasing the peptide into the solution.

In an example of production of the peptide Leu-Phe-Ala by means of the above method the support used is a synthetic resin, namely a copolymer of styrene with divinylbenzene, that forms reactive chloromethyl groups. 100 g of resin are placed into a suitable cylindrical container with attached a tube containing water condensed with CaCl₂. The resin is covered with 500 ml of anhydrous ethyl alcohol and 1.0 moles of t-butyloxycarbonylalanine is added; the mixture is refluxed for a 24-hour period in an oil bath at 90 °C.

By the subsequent step the protective group is removed by adding anhydrous hydrochloric acid (2 moles) into 20 ml of acetic acid. Then, 20 ml of triethylamine are added in order to neutralize the hydrochloric acid and to release the NH₂ radical of the amino acid which is ready, thus, to bind itself with the other amino acid, i.e. phenylalanine.

5 g of the first compound is allowed to react with 1 mole of t-butyloxycarbonylphenylalanine into an EtOH solution with the addition of NH₂-carbonyldiimidazole (500 ml); the mixture is refluxed for a 12-hour period. Subsequently the dipeptide obtained, i.e. phenylalanine-alanine, is washed with CH₂Cl₂ at 40 °C.

The compound so obtained is allowed to react with 1 mole of t-butyloxycarbonylal leucine, according to the same method and steps followed in the previous procedure. In such a way the tripeptide Leu-Phe-Ala is obtained, bound to the resin.

This product is suspended in 100 ml of trifluoroacetic acid; hydrobromic acid is bubbled through this suspension for a 2-hour period at room temperature. This reagent removes the protective t-butyloxycarbonyl groups by means of an elimination reaction, and releases the peptide from the resin by means of a reaction separating the nuclei. Washing and filtration are then performed in order to remove the solid residue made up by the resin, thus obtaining the peptide Leu-Phe-Ala in solution.

To such a solution, 20 ml of an acetate buffer solution of glacial acetic acid is added and the mixture is refluxed for half an hour at 50 °C. Finally, the solution is dried under vacuum and the desired compound, i.e. the monoacetic derivative of the tripeptide Leu-Phe-Ala, is thus obtained.

The monoacetyl-tripeptides of the present invention may be employed, either alone or in combination with each other or with other suitable active ingredients, in pharmaceutical formulations for oral or parenteral administration. Such formulations, which include pharmaceutically acceptable carriers, may be prepared by any one of the methods known in the art.

By way of example, the products of the invention may be presented in a 10% aqueous solution, so that each 3 ml vial contains 300 mg of the active ingredient.

The relevant production is carried out as follows: 30.3 g of the monoacetylpeptide, exactly weighed by means of an analytical scale, is placed in a glass beaker, and 45 ml of propylene glycol is added thereto. The mixture is heated to 80 °C in an aqueous bath, while continuously stirring. 5 ml of polyoxysorbitane monooleate (Tween 80) is then added to the mixture, and the resulting mixture is filtered through Seitz filter and is distributed into 3 ml vials. The vials are finally sterilized at 120 °C and 1 atm for 30 minutes.

The vials are subjected to all required tests prior to use, and are used in anti-cancer and/or anti-viral therapy. In particular, the product may be used to treat patients who have been subjected to surgery for a primary tumour (e.g. lung, stomach, intestine, breast, uterus tumours) and for whom there is a risk that a secondary tumour (i.e. metastasis) may develop. Further, the product may be used to treat patients who are positive to the HIV test, who are expected to develop AIDS as a further stage of the disease.

In the event that a combination of both monoacetyl-tripeptides of the invention is employed in the pharmaceutical formulation, a preferred proportion is about 50% of monoacetyl-Leu-Phe-Ala and about 50% of monoacetyl-Ala-Leu-Phe.

The monoacetic derivative of the tripeptide Leu-Phe-Ala is also named SF 078, and is referred to in the following experimental reports as P3, while the monoacetic derivative of the tripeptide Ala-Leu-Phe is also named SF 080, and is referred to below as P1.

The activity of the concerned compounds is also shown the enclosed diagrammatic representations, wherein:
figure 1 shows the effect of administration of P1 on Lewis Lung Carcinoma, in terms of weight of the tumour;
figure 2 shows the effect of administration of P1 on Lewis Lung Carcinoma, in terms of weight of the tumour and number of metastases;
figures 3 and 4 show the effect of administration of P3 on Lewis Lung Carcinoma, in terms of size of the tumour; and

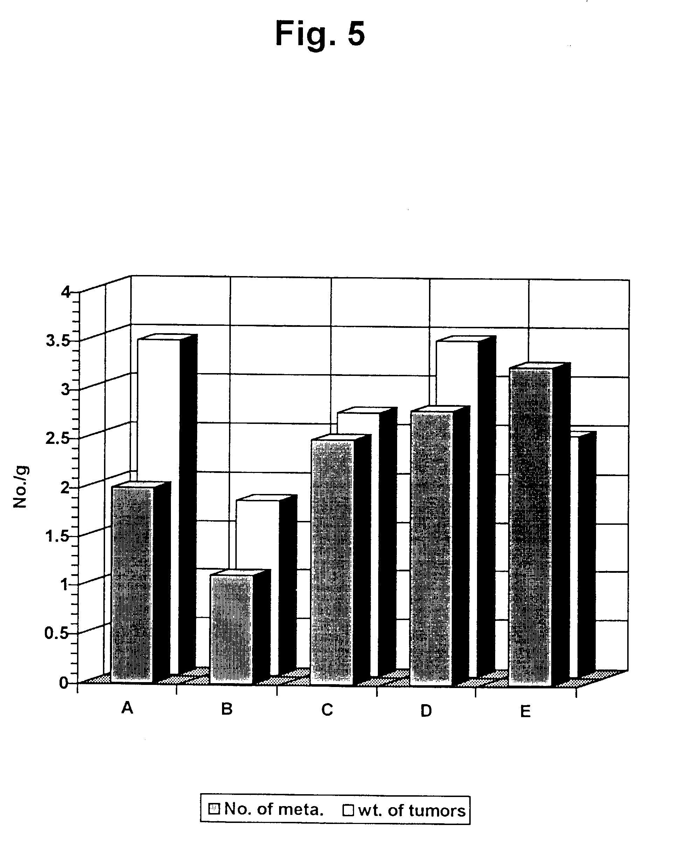

figure 5 shows the effect of administration of P3 on Lewis Lung Carcinoma, in terms of weight of the tumour and number of metastases.

### Activation test for NK lymphocytes assessed by means of rosette formation

In carrying out such a test, all six isomers of the monoacetic derivative of the peptide were used, individually, both in normoergic (germ free) animals (lot A) and in animals previously immunized with muramyl dipeptide (lot B). The method and results of the test are hereinafter reported.

### Method

A number of groups, each consisting of 18 randomized animals, were inoculated with 0.1 ml/each of normal saline solution (controls) or 0.1 ml/each of monoacetic peptide in its 6 possible isomers dissolved in normal saline solution. The 17.5 mg/kg dose administered to the animals corresponds to 1/20 of the LD found with respect to these peptides.

Treated and control animals were sacrificed in groups of 6 on the 11th, 13th and 18th day after the inoculation of the normal saline solution or of the peptide.

Afterwards, a number of groups, each consisting of 12 randomized animals, were inoculated as previously described and then all of them - treated and control animals - were sacrificed on the 13th day following the inoculation.

The lymphocytic populations studied were obtained from peripheral venous blood, withdrawn by means of intracardiac punctures from both treated and control mice.

Lymphocytes were separated according to WHO's guidelines (Aiuti et al., 1975), through a gradient on Ficoll Sodium Metr. (FCS) and subsequent washings in HBSS, and were then adjusted to the desired concentration of 4 x 10/ml.

Cell survival was assessed by means of the supravital staining technique with trypan blue. Ram's erythrocytes for rosette formation were obtained from commercially available preparations, at a 5% concentration. Following 3 serial washings in HBSS, the red blood cells were adjusted to a 0.5% concentration. Rosettes E formation was obtained by adding 0.25 ml of 0.5% ram's erythrocytes to 0.25 ml of a lymphocytes suspension at a concentration of 4 x 10/ml.

FCS, at a 10% ratio, was added to the above mentioned suspension. Following 5 minutes of incubation at +37 °C and 5 minutes of centrifugation at 200 rpm, the samples were incubated at +4 °C for 18 hours prior to reading the results.

The lymphocytes encircled by 3 or more ram's erythrocytes were considered as rosette-forming lymphocytes. The significance was assessed by means of Student's "T" test.

### Test results

Lot A: (normoergic germ free animals). The animals were tested by using the same dosage (17.5 mg/Kg) of each peptide as well as the same experimental method. The animals used were female mice belonging to BALB/C strain. In such a strain the mean of rosette-forming lymphocytes is found to be between 1% and 4% in controls. Increments in the number of rosette-forming lymphocytes below a value of a 100% increase were not taken into consideration, inasmuch as they were deemed to be poorly significant.

On the basis of such a criterion, one isomer of the peptide showed to be able to bring about, with respect to controls, a 100% increase in rosette-forming lymphocytes, and consequently to induce a statistically significant increment in the activity of the above mentioned lymphocytes. The isomer in question is the monoacetic derivative of the peptide Leu-Phe-Ala, i.e. P3

Lot B: (animals previously immunized with muramyl dipeptide). Materials and methods are the same as those used in Lot A; also the animals used were female mice belonging to BALB/C strain.

The test performed on this lot has substantiated and evidenced, to a greater extent, the activity of compound Leu-Phe-Ala, that had shown a statistically significant 100% increase when tested in Lot A animals. In fact, while in controls the preventive immunization with muramyl dipeptide brings about a significant decrement in rosette formation (and this finding appears to be in agreement with that reported in the literature: "Synthetic Adjuvans" by Arlette Adam in: Modern Concepts in Immunology, Vol. 1 - John Wiley and Sons), the animals treated with the monoacetic peptide Leu-Phe-Ala showed, under these experimental conditions, an increase in rosette formation greater than 200%.

### Activity of the peptides with respect to the proliferation processes in parenchymal organs

In order to investigate whether and how much the peptide Leu-Phe-Ala (P3) would be able to stimulate the production of proteins (immunoantibodies) in the pertinent organs (such as the spleen, etc.) and to perform consequently as an immunomodulator, some tests were carried out by using tritiated thymidine in the organs of mice belonging to BALB/C strain.

The tests have evidenced that no significant effects on the studied organs were observed in the animals examined. Therefore, the molecule investigated failed to bring about any production of immunoantibodies; thus it cannot be considered as an immunomodulator.

### Remarks on the previous two tests

The tests carried out in a lymphocytic subpopulation have shown to be highly significant with respect to the activity of one of the isomers of the peptide studied, both in the absence and in the presence of antibodies. Such an isomer is the monoacetyl Leu-Phe-Ala (i.e. P3).

On the contrary, the tests carried out in order to assess the possible production of antibodies by the above mentioned peptide were found to be negative, thus substantiating the hypothesis that the monoacetic derivative of the peptide Leu-Phe-Ala acts in a direct way on the lymphocytic subpopulation studied without the need for such lymphocytes to identify in advance any definite antigen and then to get in the cycle of the immune process.

### Tests on Lewis Lung Carcinoma

This type of solid neoplasm, designated as Lewis Lung Carcinoma, was used as a model inasmuch as it is a highly aggressive tumour; in fact, it kills 100% of the animals in which it is implanted and metastasizes by spreading from its primary site of implantation, that is the subcutaneous tissue of the animal's leg, to the lungs in 100% of the cases.

### First set of experiments

The following parameters of such an experimental model must be studied:
1) number of mice developing the neoplasm
2) number of mice with metastases
3) mean weight of primary tumour (g ± S.E.)
4) mean weight of metastases (mg ± S.E.)

Statistical analysis by means of Student's "T" test.

### Outline of the tests performed

| | | |
|---|---|---|
| 1) | time 0 | injection of peptide Leu-Phe-Ala (P3) (17.5 mg/Kg) |
| 2) | time day 13 | implantation of tumour (Lewis Lung Carcinoma 2.10x5 cells) in mice's leg - at this time the peptide shows its maximum activity |
| 3) | time day 14 | new injection of peptide Leu-Phe-Ala (P3) (same dose) |

### Results

A) All controls have developed the primary tumour and those which had not been sacrificed on the 25th day following implantation in order to verify the metastases died between the 25th and the 40th day after the implantation.
B) Among the animals that had received the peptide, an average amount of 20% survived whereas the remainder showed a mortality time that was longer than that of controls, and the difference was statistically significant (p<5. 10x⁻⁴).
C) The number of mice developing metastases was found to be 30% lower, on the average, in the animals receiving the peptide in comparison with controls.
D) In peptide treated animals the mean weight of the neoplasm was found to be 30% lower in comparison with controls (4.2 g and 6 g, respectively).
E) In treated animals the mean weight of the metastases was found to be 70% lower, on the average, in comparison with controls (35 mg and 120 mg, respectively).

### Second set of experiments

The tested compound was Ala-Leu-Phe = P1 (SF 080), again in the experimental model of tumour disease caused by Lewis Lung Carcinoma.

Animals: female mice C57Bl/6 (Charles River) having an average weight of 20g were used. The experiment involved overall 60 mice.

Tumour cells have been obtained from the third passage of Lewis Lung Carcinoma. The total number of tumour cells injected was 2 x 10⁵ in 0.1 ml of Hanks solution. The cells were injected subcutaneously into the axillary region.

The peptide (17.5 mg/kg) was administered i.p. in suspension in propylenglycol (0.1 ml/50 ml) in physiological solution. The animals of the control group, which did not receive the peptide, were injected with propylenglycol in physiological solution (0.1 ml/50 ml). The same way of administration (i.p.) was used.

The animals were divided into 5 groups each comprising 10 mice, and treated as follows.

| | | |
|---|---|---|
| Group A (control) | day-7 | control solution |
| | day 0 | control solution + tumour cells |
| | day 7 | control solution |
| Group B (3 x P1) | day -7 | peptide P1 injection |
| | day 0 | peptide P1 injection + tumour cells |
| | day 7 | peptide P1 injection |
| Group C (2 x P1) | day -7 | - |
| | day 0 | peptide P1 injection + tumour cells |
| | day 7 | peptide P1 injection |
| Group D (1 x P1) | day -7 | - |
| | day 0 | peptide P1 injection + tumour cells |
| | day 7 | - |
| Group E (2 x P1) | day -7 | peptide P1 injection |
| | day 0 | peptide P1 injection + tumour cells |
| | day 7 | - |

In this experiment the animals were daily checked for tumour growth and in view of the evident difference shown by group B the experiment was terminated 12 days after tumour injection. Mice were sacrificed, tumours were taken out and weighed.

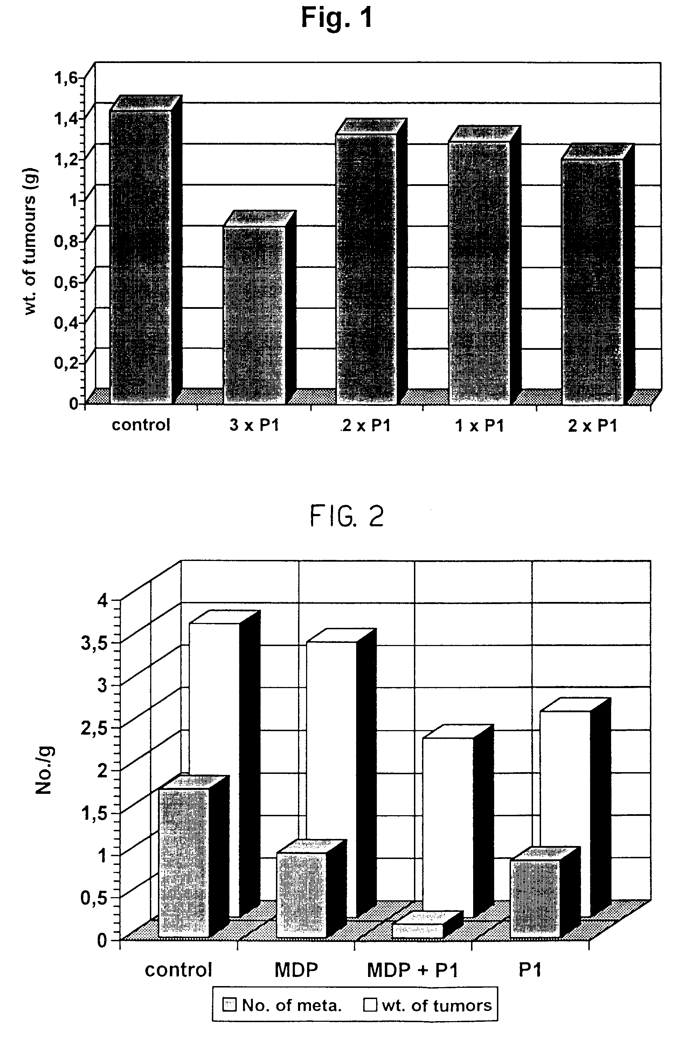

The experimental results, which are shown by the following Table and by the corresponding Figure 1, were evaluated statistically by Dunnett's and Scheffe'-test. As it can be seen from said results, in animals of group 8, where the peptide derivative P1 has been administered in 3 doses (according to the following protocol: day -7, day 0 and day 7) statistically significant results have been obtained.

**TABLE 1**

| EFFECT OF P1 (17.5 mg/kg i.p) ON LEWIS LUNG CARCINOMA | | | | | |
|---|---|---|---|---|---|
| Weight of tumour (g) | | | | | |
| animal No. | A control | B 3 x P1 | C 2 x P1 | D 1 x P1 | E 2 x P1 |
| 1 | 1.53 | 0.9 | 1.67 | 1.48 | 1.17 |
| 2 | 1.51 | 0.89 | 0.6 | 1.61 | 1.37 |
| 3 | 1.02 | 1.08 | 1.48 | 1.7 | 1.53 |
| 4 | 1.64 | 0.75 | 0.96 | 0.98 | 1.77 |
| 5 | 1.8 | 0.66 | 1.27 | 1.25 | 0.82 |
| 6 | 1.1 | 1.44 | 1.58 | 1.04 | 1.18 |
| 7 | 1.66 | 0.85 | 1.64 | 1.22 | 1.32 |
| 8 | 1.44 | 0.63 | 0.85 | 1.07 | 0.88 |
| 9 | 1.64 | 0.8 | 1.69 | 1.35 | 0.63 |
| 10 | 1.05 | 0.75 | 1.51 | 1.2 | 1.37 |
| SUM | 14.39 | 8.75 | 13.25 | 12.9 | 12.04 |
| AVG | 1.439 | 0.875 | 1.325 | 1.29 | 1.204 |
| SEM | 0.089 | 0.075 | 0.123 | 0.077 | 0.109 |
| Dunnett | - | ** | NS | NS | NS |
| Scheffe | - | ** | NS | NS | NS |

### Third set of experiments

The tested compound was Ala-Leu-Phe = P1 (SF080) (17.5 mg/kg) in the model of tumour disease caused by Lewis Lung Carcinoma. The compound was also evaluated in combination with muramyl dipeptide (MDP).

Animals: female mice C57BI/6 (Charles River) the average weight of which was 20g were used. The experiment involved overall 48 mice.

The tumour cells of LLC were obtained from the fourth passage of tumour cells. The total number of cells was 2 x 10⁵ in 0.1 ml of Hanks solution. The cells were injected subcutaneously into axillary region.

The peptide P1 was administered i.p. in suspension with propylenglycol (0.1 ml/50 ml) in physiological solution. The animals of the control group, which did not receive the peptide, were injected with propylenglycol in physiological solution (0.1 ml/50 ml). The same way of administration as in tested animals (i.p.) was used.

The animals were divided into 4 groups each having 12 mice. In groups 2 and 3 MDP was administered subcutaneously in a dose of 100 µg (0.5 ml physiol. solution per mouse). The protocol of the test is as follows.

| | | |
|---|---|---|
| Group 1 (control) | day -13 | control solution |
| | day -3 | physiological solution |
| | day 0 | tumour cells |
| Group 2 (MDP) | day -13 | - |
| | day -3 | MDP injection |
| | day 0 | tumour cells |
| Group 3 (MDP + P1) | day -13 | peptide P1 injection |
| | day -3 | MDP injection |
| | day 0 | tumour cells |
| Group 4 (P1) | day -13 | peptide P1 injection |
| | day -3 | - |
| | day 0 | tumour cells |

The animals in all groups were observed daily for tumour growth. In the control group the tumour growth was so rapid and the animals were in such a condition that the experiment was terminated after 18 days from the administration of tumour cells. Mice were sacrificed and tumour mass was excised and weighed. Both lobes of lungs were inspected for the presence of metastases.

The following parameters have been evaluated in this experiment:
a) the percentage of animals not having the tumour
b) the average weight of the tumour
c) the percentage of animals with metastases in lungs
d) the average number of metastases in case of positive animals

The results are summarized in the following Table 2, as well as in figure 2.

Statistical analysis was performed by the method of one factorial analysis of variance (ANOVA), Dunnett's test and chi-square test. The ANOVA method applied to the tumour weight led to the conclusion that differences among the tested groups are statistically significant, while the Dunnett's test confirmed that tumour weights in groups 3 and 4 are statistically significantly lower. The chi-square method confirmed that the frequency of metastases is significantly decreased in groups 3 and 4 as compared to controls.

**TABLE 2**

| EFFECT OF P1 (17.5 mg/kg i.p) ON LEWIS LUNG CARCINOMA | | | | | | | | |
|---|---|---|---|---|---|---|---|---|
| Weight of tumours (g) and average number of metastases | | | | | | | | |
| animal No. | control | | MDP | | MDP + P1 | | P1 | |
| | weight | meta | weight | meta | weight | meta | weight | meta |
| 1 | 2.67 | 4 | 3.57 | 2 | 3.67 | 0 | 3.53 | 0 |
| 2 | 4.53 | 3 | 2.72 | 0 | 1.14 | 0 | 0.47 | 0 |
| 3 | 0.5 | 0 | 1.81 | 3 | 0.81 | 0 | 3.77 | 3 |
| 4 | 3.25 | 4 | 4.11 | 0 | 2.95 | 0 | 3.09 | 0 |
| 5 | 4.04 | 3 | 2.49 | 0 | 2.62 | 1 | 2.26 | 4 |
| 6 | 1.93 | 0 | 0 | 0 | 3.95 | 4 | 0.62 | 2 |
| 7 | 4.59 | 3 | 4.88 | 1 | 0 | 0 | 2.14 | 0 |
| 8 | 4.34 | 1 | 4.48 | 3 | 1.19 | 0 | 4.55 | 0 |
| 9 | 3.39 | 0 | 1.97 | 0 | 0.28 | 0 | 3.4 | 1 |
| 10 | 4.6 | 0 | 3.13 | 0 | 0.95 | 0 | 1.2 | 0 |
| 11 | 2.97 | 1 | 0 | 0 | 2.54 | 0 | 1.6 | 0 |
| 12 | 4.55 | 2 | - | - | 3.16 | 0 | 0 | 0 |
| SUM | 41.36 | 21 | 29.16 | 9 | 23.26 | 5 | 26.63 | 10 |
| AVG | 3.45 | 1.75 | 3.24 | 1 | 2.11 | 0.16 | 2.42 | 0.91 |
| SEM | 0.37 | | 0.37 | | 0.38 | | 0.41 | |
| tumour-free | 0% | | 18% | | 8% | | 8% | |
| Dunnett | comp. | | 0.70 | | 4.47 | | 3.43 | |
| | | | NS | | ** | | ** | |
| metastase free | | 33.3 % | | 64% | | 83% | | 67% |
| chi-sq. | | | | 3.50 | | 8.40 | | 4.17 |
| | | | | NS | | ** | | ** |

### Fourth set of experiments

The tested compound was Leu-Phe-Ala = P3 (SF078) (17.5 mg/kg) in the model of tumour disease caused by Lewis Lung Carcinoma.

Animals: female mice C57Bl/6 (Charles River) the average weight of which was 20g were used. The experiment involved overall 60 mice.

The tumour cells of LLC were obtained from the twelfth passage of tumour cells. The total number of cells was 2 x 10⁵ in 0.1 ml of Hanks solution. The cells were injected subcutaneously into axillary region.

The peptide P3 was administered i.p. in suspension with propylenglycol (0.1 ml/50 ml) in physiological solution. The animals of the control group, which did not receive the peptide, were injected with propylenglycol in physiological solution (0.1 ml/50 ml). The same way of administration as in tested animals (i.p.) was used.

The animals were divided into 5 groups each having 12 mice. In groups 2 and 3 MDP was administered subcutaneously in a dose of 100 µg (0.5 ml physiol. solution per mouse). The protocol of the test is as follows.

| | | |
|---|---|---|
| Group A (control) | day -7 | control solution |
| | day 0 | control solution + tumour cells |
| | day 7 | control solution |
| Group B (3 x P3) | day -7 | peptide P3 injection |
| | day 0 | peptide P3 injection + tumour cells |
| | day 7 | peptide P3 injection |
| Group C (2 x P3) | day -7 | peptide P3 injection |
| | day 0 | peptide P3 injection + tumour cells |
| | day 7 | - |
| Group D (1 x P3) | day -7 | - |
| | day 0 | peptide P3 injection + tumour cells |
| | day 7 | - |
| Group E (2 x P3) | day -7 | - |
| | day 0 | peptide P3 injection + tumour cells |
| | day 7 | peptide P3 injection |

The animals were sacrificed on day 18 after tumour injection. The tumour mass was excised and weighed. Lungs were inspected for the presence of metastases. Those animals with obviously no tumour were left alive, and sacrificed 10 days later (i.e. on day 28). In one of these mice the tumour was found (in the group E).

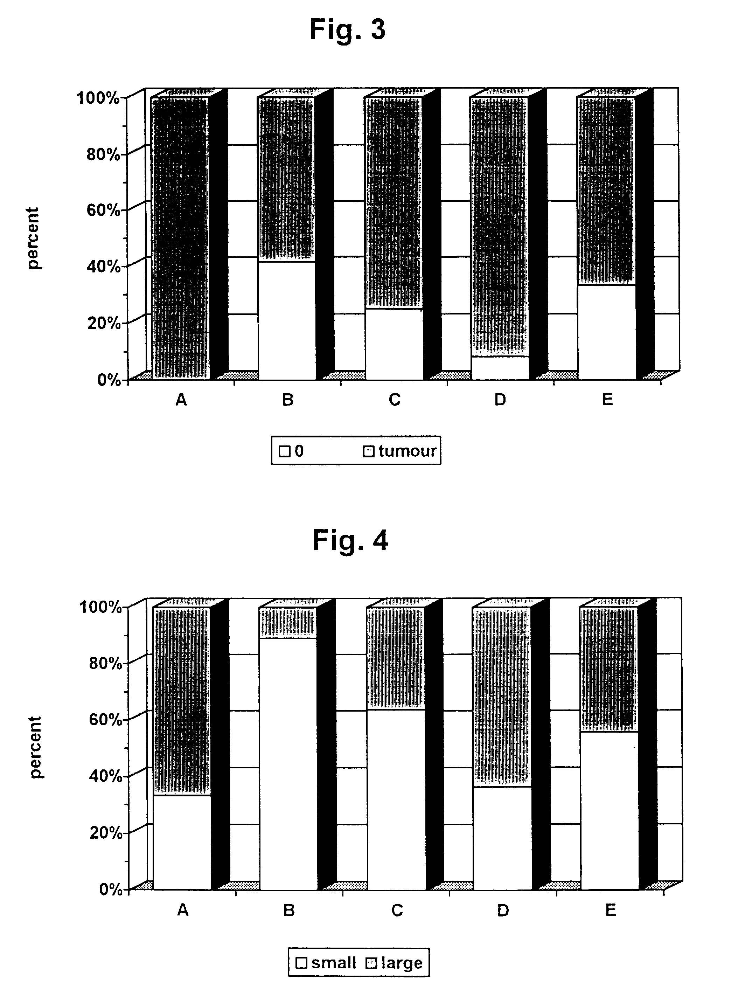

The following Table 3 and the enclosed figures 3 and 4 show the evaluation of tumour size on days 10 and 15 of the test. In the first section thereof, "0" stands for "not visible or palpable tumour", while "tumour" corresponds to cases where a tumour has been detected. In the second section of the table, i.e. after 5 more days, the distinction is made between mice showing a tumour of a small size and those showing a larger tumour. Differences in frequency of these classes were evaluated by means of the chi-square test.

**TABLE 3**

| EFFECT OF P3 (17.5 mg/kg i.p) ON LEWIS LUNG CARCINOMA | | | | | | | | | | |
|---|---|---|---|---|---|---|---|---|---|---|
| Evaluation of primary tumour size | | | | | | | | | | |
| | A control | | B 3 x P3 | | C 2 x P3 | | D 1 x P3 | | E 2 x P3 | |
| | wt | meta | wt | meta | wt | meta | wt | meta | wt | meta |
| 1 | ex day 1 | | 0.2 | 0 | 3.7 | 1 | ex day 1 | | ex day 1 | |
| 2 | 2.5 | 1 | 1 | 1 | 3.3 | 3 | 2.8 | 2 | 3.8 | 1 |
| 3 | 3.9 | 2 | 3.3 | 4 | 3 | 4 | 3.1 | 3 | 0.9 | 1 |
| 4 | 3.6 | 2 | 0.4 | 1 | 3.4 | 4 | 3.9 | 1 | 3.7 | 0 |
| 5 | 5.7 | 1 | 1.9 | 1 | 3.2 | 4 | 3 | 1 | 2.5 | 12 |
| 6 | 4.1 | 2 | 4.1 | 1 | 2.9 | 1 | 4.3 | 2 | 3.6 | 3 |
| 7 | 2.7 | 3 | 3.9 | 3 | 0.3 | 1 | 3.3 | 2 | 2.1 | 7 |
| 8 | 2.5 | 3 | 0.1 | 0 | 3.4 | 2 | 3.1 | 6 | 0.9 | 1 |
| 9 | 2.5 | 0 | 2.9 | 0 | 4.2 | 4 | 4.2 | 2 | 2.2 | 1 |
| 10 | 3.1 | 2 | 0.1 | 0 | 2.5 | 5 | 3.7 | 4 | tumour-free | |
| 11 | 3.5 | 2 | tumour-free | | 2.3 | 1 | 2.9 | 5 | tumour-free | |
| 12 | 3.5 | 4 | tumour-free | | 0.1 | 0 | tumour-free | | tumour-free | |
| AVG | 3.42 | 2 | 1.79 | 1.1 | 2.69 | 2.5 | 3.43 | 2.8 | 2.46 | 3.25 |
| SEM | 1.79 | 0.33 | 0.52 | 0.43 | 0.37 | 0.48 | 0.17 | 0.53 | 0.42 | 1.47 |
| Dunnett | - | - | ** | NS | * | NS | NS | NS | ** | NS |
| tumour-free | 0 | 0% | 2 | 16.7% | 0 | 0% | 1 | 9.1% | 3 | 27.3% |
| tumour | 11 | 100% | 10 | 83% | 12 | 100% | 10 | 90.9% | 8 | 72.7% |
| chi-sq. | - | | 4.2 | | 0 | | 4.19 | | 6.18 | |
| P= | - | | * | | NS | | * | | * | |

### Antiviral activity tests

In order to assess the increase in NK lymphocytes' activity in animals treated with the peptide derivatives of the invention, normoergic mice of the BDF1 strain have been subjected to infection by MHV3 virus, i.e. the acute hepatitis virus.

The following aspects have been taken into account in the tests.
1. survival time of the tested animals;
2. hepatic protection in the animals treated with the peptide derivatives.

### Survival tests upon MHV3 virus infection

The test has been carried out on two groups of mice of the BDF1 strain, of the weight of 16-18 g each.

The tested compound was Ala-Leu-Phe = P1 (SF 080), at a concentration of 3.5 g/l; the administered dose was 0.1 ml of the compound in normal saline solution.

The infection by the MHV3 strain had been preceded by a test by which the response of the BDF1 strain of mice to the liver-damaging action of the virus has been evaluated.

A dose of 50 LD₅₀ of the virus has been administered both to the animals treated with the physiological solution only (control) and to the animals treated with the peptide P1. The virus was injected on the 12th day starting from the day of administration of either the control solution or the peptide, on the grounds that the 12th day corresponds to the maximum activity of the NK lymphocytes, as shown by the tests carried out with the peptide only.

The following table shows the survival times in the two groups of animals (calculated by the harmonic average).

**TABLE 4**

| EFFECT OF P1 ON MHV3 VIRUS INFECTIONS Survival times (days) | | |
|---|---|---|
| | control MHV3 only | peptide P1 + MHV3 |
| 1st test | 2.39 | 9.21 |
| 2nd test | 3.61 | 14.00 |
| AVG | 3.03 | 6.24 |

### Hepatic protection

Hepatic transaminases: GOT and GPT were measured on day 5 from the infection with MHV3 virus, as this is the time of maximum infective power of the concerned virus.

The average value of hepatic transaminases in mice previously treated with P1 and then with the virus is about 50% lower than that of mice of the control group.

Autopsy of the mice's livers: after the mice's death their livers have been taken out and from sections thereof a total of 200 histologic preparations have been obtained.

Upon examination of the samples a clear difference has been evidenced between the animals belonging to the control panel and those treated with the compound of the invention. The livers of the treated animals do not show any histologic sign of any damage, while remarkable damages may be seen in the livers of the control animals, clearly resulting from the action of the virus.

## Claims

1. Monoacetic derivative, i.e. N-monoacetylated amide, of a tripeptide chosen between leucine-phenylalanine-alanine and alanine-leucine-phenylalanine.

2. Derivative according to claim 1, which is the monoacetic derivative of the tripeptide leucine-phenylalanine-alanine.

3. Derivative according to claim 1, which is the monoacetic derivative of the tripeptide alanine-leucine-phenylalanine.

4. Monoacetic derivative, i.e. N-monoacetylated amide, of a tripeptide chosen between leucine-phenylalanine-alanine and alanine-leucine-phenylalanine, for use in the fight against tumours and viruses.

5. Derivative according to claim 4, which is the monoacetic derivative of the tripeptide leucine-phenylalanine-alanine.

6. Derivative according to claim 4, which is the monoacetic derivative of the tripeptide alanine-leucine-phenylalanine.

7. Derivative according to any one of claims 4-6, for use as an anti-cancer agent.

8. Derivative according to any one of claims 4-6, for use against metastatic neoplastic cells.

9. Derivative according to any one of claims 4-6, for use as an anti-viral agent.

10. Pharmaceutical composition comprising as active ingredient the monoacetic derivative, i.e. the N-monoacetylated amide, of a tripeptide chosen between leucine-phenylalanine-alanine and alanine-leucine-phenylalanine or a mixture thereof, in a pharmaceutically acceptable carrier.

11. Pharmaceutical composition according to claim 10, for the treatment or prophylaxis of cancer and of viral diseases.

## Patentansprüche

1. Monoacetisches Derivat, das heisst N-monoacetylierte Amide, eines zwischen Leucin-Phenylalanin-Alanin und Alanin-Leucin-Phenylalanin gewählten Tripeptids.

2. Derivat nach Anspruch 1, das das monoacetische Derivat des Tripeptids Leucin-Phenylalanin-Alanin ist.

3. Derivat nach Anspruch 1, das ein monoacetisches Derivat des Tripeptids Alanin-Leucin-Phenylalanin ist.

4. Monoacetisches Derivat, das heisst N-monoacetylierte Amide eines zwischen Leucin-Phenylalanin-Alanin und Alanin-Leucin-Phenylalanin gewählten Tripeptids zur Verwendung bei der Bekämpfung von Tumoren und Viren.

5. Derivat nach Anspruch 4, das ein monoacetisches Derivat des Tripeptids Leucin-Phenylalanin-Alanin ist.

6. Derivat nach Anspruch 4, das ein monoacetisches Derivat des Tripeptids Alanin-Leucin-Phenylalanin ist.

7. Derivat nach jeglichem Anspruch zwischen 4-6 zur Verwendung als Krebsbekämpfungsagent.

8. Derivat nach jeglichem Anspruch zwischen 4-6 zur Verwendung gegen metastatische neoplastische Zellen.

9. Derivat nach jeglichem Anspruch zwischen 4-6 zur Verwendung als Antivirusagent.

10. Pharmazeutische Zusammensetzung, die als Wirkstoff das monoacetische Derivat enthält, das heisst die N-monoacetylierten Amide eines zwischen Leucin-Phenylalanin-Alanin und Alanin-Leucin-Phenylalanin oder einer Mischung davon gewählten Tripeptids in einem pharmazeutisch akzeptablen Träger.

11. Pharmazeutische Zusammensetzung nach Anspruch 10 zur Behandlung von Krebs und Viruserkrankungen oder zur Vorbeugung gegen dieselben.

## Revendications

1. Dérivé monnoacétique, à savoir amide N-monoacétylé, d'un tripeptide choisi entre leucine-phénylalanine-alanine et alanine-leucine-phénylanine.

2. Dérivé conforme à la revendication 1, qui est le dérivé monoacétique du tripeptide leucine-phénylalanine-alanine.

3. Dérivé conforme à la revendication 1, qui est le dérivé monoacétique du tripeptide alanine-leucine-phénylanine.

4. Dérivé monoacétique, à sovoir amide N-monoacétylé, d'un tripeptide choisi entre leucine-phénylalanine-alanine et alanine-leucine-phénylanine, à utiliser dans la lutte contre les tumeurs et les virus.

5. Dérivé conforme à la revendication 4, qui est le dérivé monoacétique du tripeptide leucine-phénylalanine-alanine.

6. Dérivé conforme à la revendication 1, qui est le dérivé monoacétique du tripeptide alanine-leucine-phénylanine.

7. Dérivé conforme à l'une quelconque des revendications 4-6, à utiliser comme un agent anticancer.

8. Dérivé conforme à l'une quelconque des revendications 4-6, à utiliser contre les cellules néoplasiques métastatiques.

9. Dérivé conforme à l'une quelconque des revendications 4-6, à utiliser comme un agent antiviral.

10. Composé pharmaceutique comprenant comme ingrédient actif le dérivé monoacétique, à savoir amide N-monoacétylé, d'un tripeptide choisi entre leucine-phénylalanine-alanine et alanine-leucine phénylanine ou un mélange des deux, dans une capsule acceptable pharmaceutiquement.

11. Composé pharmaceutique conforme à la revendication 10, pour le traitement ou la prophylaxie du cancer et des maladies virales.
